(19) Europäisches Patentamt
European Patent Office
Office européen des brevets

(11) EP 0 835 688 B1

(12) **FASCICULE DE BREVET EUROPEEN**

(45) Date de publication et mention de la délivrance du brevet:
**18.02.2004 Bulletin 2004/08**

(51) Int Cl.⁷: **B01J 29/04**, C07C 239/10

(21) Numéro de dépôt: **97402269.1**

(22) Date de dépôt: **29.09.1997**

(54) **Catalyseur à base de titano-silicalites et procédé d'obtention d'hydroxylamine, n,n-disubstituée**

Katalysatoren auf Basis von Titansilikaliten und Verfahren zur Herstellung von n,n-disubstituierten Hydroxylaminen

Titanium silicalites catalysts and n,n-disubstituted hydroxylamine preparation process

(84) Etats contractants désignés:
**BE CH DE ES FR GB IT LI NL**

(30) Priorité: **11.10.1996 FR 9612459**

(43) Date de publication de la demande:
**15.04.1998 Bulletin 1998/16**

(73) Titulaire: **Atofina**
**92800 Puteaux (FR)**

(72) Inventeurs:
- **Teissier, Rémy**
**69340 Francheville (FR)**
- **Jorda, Eric**
**69001 Lyon (FR)**

(74) Mandataire: **Granet, Pierre**
**Atofina,**
**Département Propriété Industrielle,**
**4-8, cours Michelet,**
**La Défense 10**
**92091 Paris La Défense Cedex (FR)**

(56) Documents cités:

| | |
|---|---|
| **EP-A- 0 522 634** | **EP-A- 0 655 278** |
| **EP-A- 0 665 188** | **DE-A- 4 435 239** |
| **FR-A- 2 471 950** | **US-A- 4 938 939** |

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne un procédé d'obtention d'hydroxylamine, N, N-disubstituée à partir du peroxyde d'hydrogène et de l'amine disubstituée correspondante en présence d'un catalyseur à base de titano-silicalites ayant une teneur pondérale en métal ou métaux alcalin(s) comprise entre 0,05 et 2 %.

On entend par titano-silicalites, des zéolithes de type MFI dont une partie des atomes de silicium est substituée par des atomes de titanes. Ces titano-silicalites ont fait l'objet de nombreuses publications. Ainsi, dans le brevet américain US 4 410 501, est décrit une méthode de préparation des titano-silicalites par réaction d'une source de silicium avec une source d'oxyde de titane, en milieu aqueux, à une température comprise entre 130 et 200° C, sous pression autogène et en présence d'une base azotée telle qu'un hydroxyde d'ammonium quaternaire. Dans l'exemple 1 de ce brevet, il est précisé que l'hydroxyde d'ammonium quaternaire doit être exempt d'ion alcalin.

D'après certains auteurs, en particulier B. NOTARI ("Structure-Activity and Selectivity Relationships in Heterogeneous Catalysis", Elsevier Science Publishers 1991 p. 248) et G. Bellussi et V. Fattore ("Zeolite Chemistry and Catalysis", Elsevier Science Publishers, 1991 p. 79), la synthèse des titano-silicalites nécessite l'utilisation des réactifs exempt d'impuretés, notamment les métaux alcalins. Ces auteurs ont montré qu'en présence de métaux alcalins, la réaction entre une source de silicium et un dérivé hydrolysable du titane comme les titanates d'alkyles, ne conduit pas à la formation des titano-silicalites.

De plus, les titano-silicalites préparés selon le brevet US 4 410 501 doivent subir plusieurs étapes d'activation avec le peroxyde d'hydrogène et un acide fort, avant de pouvoir être utilisés comme catalyseur (EP 208 311, EP 267 362 et EP 314 147).

Par ailleurs, la demande européenne EP 665 188 divulgue un procédé d'obtention de titano-silicalites à partir d'une source de silicium et du $TiF_4$ et éventuellement en présence de métal ou métaux alcalin(s). Les titano-silicalites ainsi préparés, sont lavés à l'acide chlorhydrique avant d'être utilisés dans la réaction d'hydroxylation du phénol.

La demanderesse a maintenant découvert un catalyseur à base de titano-silicalites, qui est à la fois actif et sélectif dans des réactions d'oxydation mettant en oeuvre du peroxyde d'hydrogène.

Ce catalyseur offre une sélectivité par rapport à l'eau oxygénée, plus élevée que ceux de l'art antérieur. De plus, son procédé d'obtention a l'avantage de ne pas présenter les inconvénients cités précédemment.

Le catalyseur, est à base de titano-silicalites ayant une teneur pondérale en métal ou métaux alcalin(s) comprise entre 0,05 et 2 %, de préférence comprise entre 0,9 et 1,5 %. Il peut être préparé par traitement hydrothermal d'un mélange réactionnel constitué d'une source de silicium tétravalent, du tétrafluorure de titane ($TiF_4$), d'un agent structurant, de l'eau et d'un ou de plusieurs ion(s) alcalin.

Comme source de silicium on peut faire appel aux dérivés du silicium utilisés habituellement tels que : les silices finement divisées sous forme d'hydrogels, d'aérogels ou de suspensions colloïdales ; les esters siliciques hydrolysables comme les orthosilicates d'alkyle de formule $Si(OR)_4$ dans laquelle R représente un radical alkyle comportant de un à quatre atomes de carbone (radicaux méthyle, éthyle, propyles, butyles). On fait appel de préférence à l'orthosilicate de tétraéthyle.

L'agent structurant peut être choisi parmi ceux proposés antérieurement et notamment parmi les hydroxydes de tétraalkylammonium dans lesquels les groupes alkyles comportent de 1 à 4 atomes de carbone ; on fait appel de préférence aux hydroxydes de tétrapropylammonium et de tétrabutylammonium. Comme il n'est pas nécessaire de recourir à des hydroxydes d'ammoniun quaternaires débarrassés des ions alcalins qu'ils contiennent, des solutions aqueuses commerciales d'hydroxydes d'ammonium quaternaire peuvent convenir.

Le ou les ion(s) alcalin(s) peut ou peuvent provenir de l'agent structurant et/ou par ajout soit sous forme d'hydroxyde soit sous forme de sel. L'ion alcalin peut être choisi parmi le potassium, le sodium et le césium et de préférence parmi le potassium et le sodium.

Les conditions de la réaction hydrothermale sont celles habituellement utilisées. Le procédé se déroule en deux phases. Au cours d'une première phase on prépare un mélange réactionnel aqueux contenant au moins une source de silicium, l'agent structurant, le ou les ions alcalins et le tétrafluorure de titane. Ce mélange peut être maintenu à une température comprise entre environ 15° C et environ 50° C et pendant un temps suffisant pour procéder à l'hydrolyse de la source de silicium ; la durée de cette phase dépend de la température choisie. En général, une durée comprise entre environ 10 mn et 2 heures convient bien. Le pH du milieu réactionnel est de préférence supérieur à 10 ; un pH de 10 à 12 convient bien. Le mode d'addition des réactifs ne revêt aucun caractère critique ; ainsi, le tétrafluorure de titane peut être ajouté au milieu aqueux contenant la source de silicium avant ou après addition de l'agent structurant, et dans ce dernier cas, de préférence après avoir procédé à l'hydrolyse partielle de la source de silicium. Le tétrafluorure de titane peut être ajouté au milieu d'hydrolyse soit sous forme de poudre soit sous forme d'une suspension dans un liquide vecteur. Compte tenu de sa stabilité à l'hydrolyse à température ambiante, on peut utiliser l'eau comme liquide vecteur.

Au cours de la deuxième phase on réalise la cristallisation de la titano-silicalite par chauffage de la masse réactionnelle provenant de la première phase sous pression autogène, à une température allant de 120 à 200° C et,

de préférence, de 160 à 190° C. La durée de la phase de cristallisation dépend des conditions de la réaction ; en général cette durée est comprise entre 1 et 7 jours. Lorsqu'on utilise comme source de silicium un silicate d'alkyle on procède à l'élimination de l'alcool formé au cours de l'hydrolyse par distillation à pression normale ou sous pression réduite, avant de réaliser la phase de cristallisation.

Les quantités de dérivés du silicium et de $TiF_4$ exprimées en moles peuvent varier dans de larges limites en fonction de la composition désirée du titano-silicalite, c'est-à-dire du rapport Si/Ti recherché. Ces quantités peuvent être voisines de la stochiométrie de la réaction pour la composition visée ou s'en écarter sensiblement. En général, la quantité de $TiF_4$ exprimée en moles par mole de dérivé de silicium et en particulier par mole d'orthosilicate d'alkyle peut être comprise dans un intervalle d'environ 0,005 à environ 0,2 et, de préférence, d'environ 0,05 à environ 0,15.

La quantité d'agent structurant utilisée dans le procédé, exprimée en moles par mole de dérivé du silicium et notamment d'orthosilicate d'alkyle peut être comprise dans un intervalle d'environ 0,1 à environ 2 et de préférence d'environ 0,2 à environ 0,6.

La quantité d'eau présente dans le milieu réactionnel n'est pas critique et peut varier dans de larges limites. En général une quantité d'eau comprise dans un intervalle d'environ 5 à environ 100 moles et, de préférence, d'environ 20 à environ 50 moles par mole de dérivé du silicium convient bien.

Le catalyseur obtenu à la fin de la réaction est séparé du milieu réactionnel par filtration, lavé à l'eau, séché à température supérieure ou égale à 100° C puis calciné à une température supérieure ou égale à 500° C pour éliminer l'agent structurant.

La quantité de titane présent dans le catalyseur est en général comprise entre 0,6 % et 2,5 %, de préférence comprise entre 0,8 et 1,5 %.

La préparation du catalyseur, diffère principalement de celle décrite dans la demande européenne EP 665 188 par l'absence de l'étape de lavage. En effet, le lavage à l'acide est destiné à éliminer les métaux alcalins.

Le catalyseur, convient tout particulièrement pour fabriquer des hydroxylamines, N,N-disubstituées de formule générale (I):

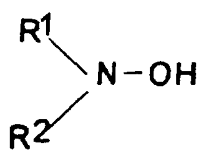

(I)

dans laquelle $R^1$ et $R^2$ identiques ou différents, représentent chacun un radical alkyle linéaire ou ramifié, de préférence linéaire, contenant de 1 à 8 atomes de carbone, de préférence de 1 à 6 atomes de carbone, éventuellement substitué, de préférence en bout de chaîne, par des groupements choisis parmi l'hydroxyle, le fluorure, les sulfonates des métaux alcalins ou alcalino-terreux, les carboxylates de métaux alcalins ou alcalino-terreux et l'éther, un radical cycloalkyle contenant de 5 à 8 atomes de carbone, un radical linéaire de formule $C_nH_{2n-1}$, n étant un nombre compris entre 2 et 6. $R^1$ et $R^2$, ensemble avec l'atome d'azote, peuvent également être reliés entre eux en formant un cycle saturé ou insaturé comprenant de 4 à 7 atomes de carbone.

L'objet de l'invention concerne donc un procédé d'obtention d'hydroxylamine, N,N-disubstituée de formule générale (I) à partir du peroxyde d'hydrogène et de l'amine correspondante. Ce procédé est caractérisé en ce que l'on opère en présence d'un catalyseur, comme décrit précédemment.

Les amines correspondantes préférées sont la diéthylamine, la dibutylamine, la dihexylamine, la méthyléthylamine, la méthylpropylamine, la méthylbutylamine, l'éthylbutylamine, l'éthylhexylamine, la diéthanolamine, la divinylamine, la piperidine, la morpholine, la pyrrolidine et la pyrroline.

Les amines correspondantes particulièrement préférées sont la diéthylamine, la diéthanolamine, la divinylamine, la méthyléthylamine, la méthyl propylamine et la méthylbutylamine.

Selon le procédé de la présente invention, on peut opérer soit en présence soit en l'absence d'un solvant. Comme solvant, on peut choisir l'eau ou tout solvant organique miscible à l'eau, par exemple les alcools aliphatiques et les éthers lourds type glyme.

Avantageusement, le tertiobutanol et le diglyme sont choisis.

Le procédé peut être conduit aussi bien en continu qu'en discontinu.

La température réactionnelle est en général comprise entre 15 et 150° C et de préférence comprise entre 60 et 90° C.

On peut opérer à la pression atmosphérique et également à une pression supérieure à la pression atmosphérique de manière à maintenir le solvant ainsi que les réactifs à l'état liquide.

Le catalyseur peut être utilisé soit à l'état finement divisé et dispersé dans le milieu réactionnel soit mis en

forme pour donner des pastilles ou extrudats. La quantité de catalyseur mise en jeu est en général comprise entre 0,5 et 50 parties pour 100 parties d'amine et de préférence comprise entre 1 et 30 parties.

Le peroxyde d'hydrogène utilisé est en général sous forme de solution aqueuse de concentration comprise entre 10 et 70 % en poids. On utilise le plus souvent une solution aqueuse de 20 à 40 % en poids de peroxyde d'hydrogène.

La quantité de peroxyde d'hydrogène mise en jeu est telle que le rapport molaire peroxyde d'hydrogène / amine est compris entre 0,2 et 1,5 et de préférence compris entre 0,9 et 1,2 lorsqu'on travaille en discontinu.

Lorsqu'on travaille en présence d'un solvant, la quantité utilisée est telle que la concentration de l'amine dans le solvant est comprise entre 1 et 30 parties pour 100 parties de solvant.

Lorsqu'on travaille en discontinu, on obtient, à la fin de la réaction, une suspension contenant le catalyseur. Ce dernier peut être récupéré par filtration de la suspension et le filtrat ainsi obtenu constitué de l'amine non réagie et des produits de la réaction, est analysé en chromatographie phase gazeuse. L'amine non réagie et l'hydroxylamine, N,N-disubstituée peuvent également être dosées par acidimétrie. Les différents produits de la réaction ainsi que l'amine non réagie peuvent être isolés du filtrat par les techniques connus telles que distillation, cristallisation ou extraction. L'amine non réagie et le solvant peuvent être recyclés.

L'invention sera mieux comprise à l'aide des exemples suivants.

## PARTIE EXPERIMENTALE

### Préparation du catalyseur

Dans un ballon de 250 $cm^3$ équipé d'un dispositif de chauffage et d'un système d'agitation, on introduit 25 $cm^3$ de silicate d'éthyle et 40 $cm^3$ d'une solution aqueuse de tétrapropylammonium hydroxyde (ALDRICH), à une mole par litre, contenant 0,1 % en poids d'ions sodium et 0,32 % en poids d'ions potassium. On démarre l'agitation, puis on maintient le mélange pendant 30 minutes à température ambiante. On y rajoute ensuite 40 $cm^3$ d'eau distillée et on porte alors le mélange à 80° C. Le mélange est maintenu pendant 2 heures à cette température. Au bout de 2 heures, on arrête le chauffage et on y ajoute 0,32 g de $TiF_4$. Le milieu réactionnel est ensuite laissé sous agitation pendant 5 minutes, puis transvasé dans un réacteur résistant à la pression, pourvu d'un revêtement intérieur en Téflon, équipé d'un système d'agitation et d'un dispositif de chauffage. Le milieu réactionnel est ensuite porté à 170° C en une heure, sous agitation (300 rotations par minute) et est maintenu ainsi pendant 2 jours. Le solide blanc formé pendant la réaction, est filtré puis lavé 5 fois avec 250 $cm^3$ d'eau distillée à chaque fois. Le solide filtré est ensuite séché à 120° C pendant 12 heures, puis calciné à 500° C sous air pendant 4 heures.

On obtient ainsi 5,3 g d'un solide blanc dont la teneur pondérale en Ti est de 0,9 %, en F = 0,07 %, en K = 0,8 % et en Na = 0,045 %.

Le produit analysé en diffraction des rayons X et en infra-rouge, donne des spectres caractéristiques des titanosilicalites. De plus, le spectre en ultraviolet indique que le produit est dépourvu de titane extra-réseau.

Dans ce qui suit, on définit la sélectivité de la diéthylhydroxylamine (DEHA) par rapport à l'amine correspondante (DEA) par la formule suivante :

$$\text{Sélectivité (DEHA/DEA)} = \frac{\text{nombre de moles de DEHA formées x 100}}{(DEA)_i - (DEA)_f}$$

où $(DEA)_i$ = nombre de moles de DEA à l'état initial
où $(DEA)_f$ = nombre de moles de DEA à l'état final

De même la sélectivité de la DEHA par rapport au peroxyde d'hydrogène ($H_2O_2$) est définie par la formule suivante :

$$\text{Sélectivité (DEHA/}H_2O_2\text{)} = \frac{\text{nombre de moles de DEHA formées x 100}}{(H_2O_2)_i - (H_2O_2)_f}$$

où $(H_2O_2)_i$ = nombre de moles de $H_2O_2$ à l'état initial
et $(H_2O_2)_f$ = nombre de moles de $H_2O_2$ à l'état final

### ***Exemple 1***

Dans un réacteur thermostaté, équipé d'un système d'agitation, d'un dispositif d'introduction de réactif liquide,

d'une prise de température et d'un condensateur à reflux relié à une cuve à eau, on introduit :

- 11 g de diéthylamine (0,15 mole)
- 60 g de tertiobutanol et
- 2 g de catalyseur préparé selon le mode opératoire ci-dessus.

On porte le mélange, sous agitation, à la température de reflux (79-80° C), puis on introduit, dans le réacteur pendant une heure, 13,1 g d'une solution aqueuse de peroxyde d'hydrogène à 35 % en poids (0,135 mole).

On arrête le chauffage, 15 minutes après la fin de l'introduction de la solution aqueuse de peroxyde d'hydrogène. La cuve à eau qui est le dispositif pour évaluer le taux de décomposition du peroxyde d'hydrogène, indique l'absence de dégagement gazeux.

Après refroidissement, le peroxyde d'hydrogène non consommé est dosé par iodométrie et la solution finale est ensuite analysée en chromatographie phase gazeuse et également dosée par acidimétrie.

Les analyses indiquent qu'à la fin de la réaction, on obtient un taux de conversion de diéthylamine (DEA) de 87 %, une sélectivité en diéthylhydroxylamine (DEHA) et en nitrone par rapport à la diéthylamine de 87 % et 11 % respectivement.

L'analyse de la solution finale montre que le peroxyde d'hydrogène a été totalement consommé et que la sélectivité en diéthylhydroxylamine et en nitrone par rapport au peroxyde d'hydrogène est de 84 % et 10 % respectivement.

***Exemple 2***

A la fin de la réaction de l'exemple 1, le catalyseur est séparé de la solution par filtration. Le catalyseur ainsi récupéré non séché, est ensuite utilisé dans les mêmes conditions que celles décrites précédemment (exemple 1).

***Exemple 3 comparatif***

On opère comme décrit à l'exemple 1 sauf que le catalyseur utilisé, a été préparé suivant la méthode décrite dans l'exemple 2 du brevet US 4 410 501.

Dans ce cas, la cuve d'eau indique la présence de dégagement gazeux lors de la réaction. Par conséquent, une partie du peroxyde d'hydrogène consommée, a été décomposée.

***Exemple 4 comparatif***

On opère comme décrit à l'exemple 1 sauf que le catalyseur utilisé, a été préparé suivant la méthode décrite dans l'exemple 2 de la demande européenne EP 665 188. Après lavage à l'acide et avant utilisation, le catalyseur a une teneur pondérale en Ti de 1,39 %, en F de 0,083 %, en K de 0,01 % et en Na de 0,02 %.

***Exemple 5 comparatif***

On opère comme décrit à l'exemple 3 sauf que le catalyseur est soumis à un prétraitement par une solution aqueuse de KCl à 0,1 mole par litre et une solution aqueuse de NaCl à 0,01 mole par litre.

Le catalyseur prétraité a une teneur pondérale en Ti de 1,04 %, en K de 0,85 % et en Na de 0,065 %.

Les résultats obtenus des exemples 1 à 5 sont reportés dans le Tableau 1.

***Exemple 6***

On mélange 80 parties en poids du catalyseur préparé selon le mode opératoire décrit dans la présente demande, avec 20 parties d'un liant à base de gel de silice (20 % en poids de silice), puis on le met en forme pour donner des extrudats de diamètre et de longueur compris entre 1 mm et 1,6 mm. Les extrudats sont ensuite calcinés à 500° C sous air pendant 4 heures.

Dans un réacteur cylindrique de diamètre intérieur 20 mm et équipé d'un fritté et d'une double paroi, thermostaté à l'aide d'un fluide thermique circulant, on introduit 12 g d'extrudats ainsi préparés. Le lit du catalyseur repose sur le fritté situé à 3 cm à partir de la partie inférieure du réacteur. Le réacteur cylindrique comporte une zone réactionnelle, constituée du lit catalytique et d'une zone de préchauffage, située juste en-dessous du lit catalytique.

| EXEMPLE | CONVERSION DEA % | SELECTIVITE/DEA % | | CONVERSION $H_2O_2$ % | SELECTIVITE/$H_2O_2$ % | | DECOMPOSITION $H_2O_2$ |
|---|---|---|---|---|---|---|---|
| | | DEHA | NITRONE | | DEHA | NITRONE | |
| 1 | 87 | 87 | 11 | 100 | 84 | 10 | NON |
| 2 | 87 | 86 | 11 | 100 | 83 | 10 | NON |
| 3 | 78 | 80 | 20 | 100 | 67 | 17 | OUI |
| 4 | 80 | 78 | 19 | 100 | 69 | 17 | OUI |
| 5 | 60 | 75 | 22 | 100 | 52 | 15 | OUI |
| 6 | 24 | 95 | 5 | 100 | 92 | 8 | NON |

Tableau 1

On introduit en continu dans le réacteur une solution de diéthylamine à 19 % en poids dans le diglyme avec un débit de 256 g/h et une solution aqueuse de peroxyde d'hydrogène à 20 % en poids avec un débit de 19,3 g/h. Avant d'être introduites dans le réacteur, les solutions sont mélangées.

La température du fluide thermique est réglée de façon à obtenir une température dans la zone réactionnelle égale à 78° C et l'ensemble de l'installation est balayé sous azote.

A la sortie du réacteur, le liquide est récupéré par surverse puis refroidi avant d'être stocké.

Les résultats reportés dans le tableau 1, sont obtenus pour une durée de fonctionnement de 3 heures.

## Revendications

1. Procédé d'obtention d'hydroxylamine N,N-disubstituée de formule générale :

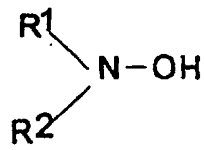

dans laquelle $R^1$ et $R^2$ identiques ou différents, représentent chacun un radical alkyle, linéaire ou ramifié, de préférence linéaire, contenant de 1 à 8 atomes de carbone, de préférence de 1 à 6 atomes de carbone, éventuellement substitué, de préférence en bout de chaîne, par des groupements choisis parmi l'hydroxyle, le fluorure, les sulfonates des métaux alcalins ou alcalino-terreux, les carboxylates de métaux alcalins ou alcalino-terreux et l'éther, un radical cycloalkyle contenant de 5 à 8 atomes de carbone, un radical linéaire de formule $C_nH_{2n-1}$, n étant un nombre compris entre 2 et 6, à partir du peroxyde d'hydrogène et de l'amine disubstituée correspondante et en présence d'un catalyseur à base de titanosilicalites, ayant une teneur pondérale en métal ou métaux alcalin(s) comprise entre 0,05 et 2 % et susceptible d'être obtenu par traitement hydrothermal d'un mélange réactionnel constitué d'une source de silicium tétravalent, de tétrafluorure de titane, d'un agent structurant, d'eau et d'un ou de plusieurs ion(s) alcalin(s).

2. Procédé selon la revendication 1 **caractérisé en ce que** la teneur pondérale en métal ou métaux alcalin(s) du catalyseur est comprise entre 0,9 % et 1,5 %.

3. Procédé selon la revendication 1 ou 2 **caractérisé en ce que** le(s) métal ou métaux alcalin(s) du catalyseur est ou sont choisi(s) parmi le potassium, le sodium et le césium.

4. Procédé selon l'une quelconque des revendications 1 à 3 **caractérisé en ce que** la source de silicium tétravalent est choisie dans le groupe formé par la silice finement divisée sous forme d'hydrogel, d'aérogel, ou de suspension colloïdale et les orthosilicates d'alkyle de formule générale : Si $(OR)_4$ dans laquelle R représente un radical alkyle ayant de 1 à 4 atomes de carbone.

5. Procédé selon la revendication 4 **caractérisé en ce que** la source de silicium est l'orthosilicate de tétraéthyle.

6. Procédé selon l'une quelconque des revendications 1 à 5 **caractérisé en ce que** l'agent structurant est un hydroxyde de tétra alkylammonium dans lequel le radical alkyle comporte de 1 à 4 atomes de carbone.

7. Procédé selon la revendication 6 **caractérisé en ce que** l'agent structurant est l'hydroxyde de tétrapropylammonium ou de tétrabutylammonium.

8. Procédé selon l'une quelconque des revendications 1 à 7 **caractérisé en ce que** la teneur pondérale en titane est comprise entre 0,6 et 2,5 %.

9. Procédé selon l'une quelconque des revendications 1 à 8 **caractérisé en ce que** la température du traitement hydrothermal est comprise entre 120 et 200° C.

10. Procédé selon l'une des revendications 1 à 9 **caractérisé en ce que** $R^1$ et $R^2$ ensemble avec l'atome d'azote peuvent être reliés entre eux en formant un cycle saturé ou insaturé comprenant de 4 à 7 atomes de carbone.

11. Procédé selon l'une des revendications 1 à 9 **caractérisé en ce que** l'amine disubstituée correspondante est la diéthylamine.

## Claims

1. Process for producing N,N-disubstituted hydroxylamine of general formula:

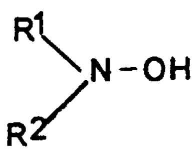

in which $R^1$ and $R^2$, which are identical or different, each represent a linear or branched, preferably linear, alkyl radical containing from 1 to 8 carbon atoms, preferably from 1 to 6 carbon atoms, which is optionally substituted, preferably at the chain end, by groups chosen from hydroxyl, fluoride, alkali metal or alkaline-earth metal sulphonates, alkali metal or alkaline-earth metal carboxylates and ether, a cycloalkyl radical containing from 5 to 8 carbon atoms or a linear radical of formula $C_nH_{2n-1}$, n being a number between 2 and 6, from hydrogen peroxide and the corresponding disubstituted amine and in the presence of a catalyst based on titanosilicalites having a content by weight of alkali metal or metals of between 0.05 and 2% which is capable of being obtained by hydrothermal treatment of a reaction mixture composed of a tetravalent silicon source, titanium tetrafluoride, a structuring agent, water and one or more alkali metal ion(s).

2. Process according to Claim 1, **characterized in that** the content by weight of alkali metal or metals of the catalyst is between 0.9% and 1.5%.

3. Process according to Claim 1 or 2, **characterized in that** the alkali metal or metals of the catalyst is or are chosen from potassium, sodium and caesium.

4. Process according to any one of Claims 1 to 3, **characterized in that** the tetravalent silicon source is chosen from the group formed by finely divided silica in the form of a hydrogel, aerogel or colloidal suspension and alkyl orthosilicates of general formula $Si(OR)_4$, in which R represents an alkyl radical having from 1 to 4 carbon atoms.

5. Process according to Claim 4, **characterized in that** the silicon source is tetraethyl orthosilicate.

6. Process according to any one of Claims 1 to 5, **characterized in that** the structuring agent is a tetraalkylammonium hydroxide in which the alkyl radical contains from 1 to 4 carbon atoms.

7. Process according to Claim 6, **characterized in that** the structuring agent is tetrapropylammonium or tetrabutylammonium hydroxide.

8. Process according to any one of Claims 1 to 7, **characterized in that** the content by weight of titanium is between 0.6 and 2.5%.

9. Process according to any one of Claims 1 to 8, **characterized in that** the temperature of the hydrothermal treatment is between 120 and 200°C.

10. Process according to one of Claims 1 to 9, **characterized in that** $R^1$ and $R^2$, together with the nitrogen atom, can be connected to one another, forming a saturated or unsaturated ring comprising from 4 to 7 carbon atoms.

11. Process according to one of Claims 1 to 9, **characterized in that** the corresponding disubstituted amine is diethylamine.

## Patentansprüche

1. Verfahren zur Herstellung eines N,N-disubstituierten Hydroxylamins der allgemeinen Formel:

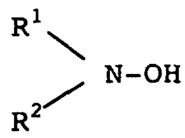

worin $R^1$ und $R^2$ gleich oder verschieden sind und jeweils für einen linearen oder verzweigten und vorzugsweise linearen Alkylrest, der 1 bis 8 Kohlenstoffatome und vorzugsweise 1 bis 6 Kohlenstoffatome aufweist und gegebenenfalls, vorzugsweise am Kettende, durch unter Hydroxyl, Fluorid, Alkalimetall- und Erdalkalimetallsulfonaten, Alkalimetall- und Erdalkalimetallcarboxylaten und Ether ausgewählte Gruppen substituiert ist, einen Cycloalkylrest mit 5 bis 8 Kohlenstoffatomen oder einen linearen Rest der Formel $C_nH_{2n-1}$, wobei n eine Zahl zwischen 2 und 6 bedeutet, stehen, aus Wasserstoffperoxid und dem entsprechenden disubstituierten Amin in Gegenwart eines Katalysators auf Basis von Titansilicaten, der einen Gehalt an Alkalimetall bzw. Alkalimetallen zwischen 0,05 und 2 Gew.-% aufweist und durch hydrothermale Behandlung einer Reaktionsmischung aus einer Quelle von vierwertigem Silicium, Titantetrafluorid, einem Strukturbildner, Wasser und einem oder mehreren Alkalimetallionen erhältlich ist.

2. Verfahren nach Anspruch 1, **dadurch gekennzeichnet, daß** der Gehalt an Alkalimetall bzw. Alkalimetallen des katalysators zwischen 0,9 und 1,5 Gew.-% liegt.

3. Verfahren nach Anspruch 1 oder 2, **dadurch gekennzeichnet, daß** man das Alkalimetall bzw. die Alkalimetalle des katalysators unter Kalium, Natrium und Cäsium auswählt.

4. Verfahren nach einem der Ansprüche 1 bis 3, **dadurch gekennzeichnet, daß** man die Quelle von vierwertigem Silicium aus der Gruppe bestehend aus feinteiligem Siliciumdioxid in Form eines Hydrogels, eines Aerogels oder einer kolloidalen Suspension und Alkylorthosilicaten der allgemeinen Formel $Si(OR)_4$, worin R für einen Alkylrest mit 1 bis 4 Kohlenstoffatomen steht, auswählt.

5. Verfahren nach Anspruch 4, **dadurch gekennzeichnet, daß** man als Siliciumquelle Tetraethylorthosilicat einsetzt.

6. Verfahren nach einem der Ansprüche 1 bis 5, **dadurch gekennzeichnet, daß** man als Strukturbildner ein Tetraalkylammoniumhydroxid mit 1 bis 4 Kohlenstoffatomen im Alkylrest verwendet.

7. Verfahren nach Anspruch 6, **dadurch gekennzeichnet, daß** man als Strukturbildner Tetrapropylammoniumhydroxid oder Tetrabutylammoniumhydroxid verwendet.

8. Verfahren nach einem der Ansprüche 1 bis 7, **dadurch gekennzeichnet, daß** der Titangehalt zwischen 0,6 und 2,5 Gew.-% liegt.

9. Verfahren nach einem der Ansprüche 1 bis 8, **dadurch gekennzeichnet, daß** man die hydrothermale Behandlung bei einer Temperatur zwischen 120 und 200°C durchführt.

10. Verfahren nach einem der Ansprüche 1 bis 9, **dadurch gekennzeichnet, daß** $R^1$ und $R^2$ zusammen mit dem N-Atom miteinander zu einem gesättigten oder ungesättigten Ring mit 4 bis 7 Kohlenstoffatomen verbunden sein können.

11. Verfahren nach einem der Ansprüche 1 bis 9, **dadurch gekennzeichnet, daß** man als entsprechendes disubstituiertes Amin Diethylamin verwendet.